(19) Europäisches Patentamt
European Patent Office
Office européen des brevets

(11) **EP 3 593 822 A1**

(12) **EUROPEAN PATENT APPLICATION**

(43) Date of publication:
**15.01.2020 Bulletin 2020/03**

(51) Int Cl.:
*A61K 49/06* (2006.01)  *A61K 33/32* (2006.01)
*A61K 31/196* (2006.01)  *A61K 31/445* (2006.01)
*A61K 31/4709* (2006.01)  *A61P 25/00* (2006.01)
*A61P 35/00* (2006.01)

(21) Application number: **18305954.2**

(22) Date of filing: **13.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Theranexus**
**69008 Lyon (FR)**

(72) Inventors:
• **DROGUERRE, Marine**
**94250 GENTILLY (FR)**
• **DUCHENE, Adeline**
**75017 PARIS (FR)**
• **MOUTHON, Franck**
**92100 BOULOGNE BILLANCOURT (FR)**
• **CHARVERIAT, Mathieu**
**92140 CLAMART (FR)**

(74) Representative: **Regimbeau**
**20, rue de Chazelles**
**75847 Paris Cedex 17 (FR)**

(54) **THERAPEUTIC AND DIAGNOSTIC APPLICATIONS OF A NEW METHOD OF MONITORING OF NEUROGLIAL INTERACTION MEMRI)**

(57) Astroglial networks have been recently considered as emerging new therapeutic targets in CNS disorders. However, the full understanding of the complex relations between astrocytes and neurons are limited due to the lack of adequate imaging tools. Here, the present inventors propose the use of Manganese Enhanced Magnetic Resonance Imaging (MEMRI) as a new tool for evaluating *in vivo* the effects of modulators of astrocyte connexins, especially connexin 30 (Cx30) and connexin 43 (Cx43) inhibitors. They also propose to combine these modulators to manganese-based compounds for inducing antitumor and radiosensitizing effects.

EP 3 593 822 A1

**Description**

**Summary**

Astroglial networks have been recently considered as emerging new therapeutic targets in CNS disorders. However, the full understanding of the complex relations between astrocytes and neurons are limited due to the lack of adequate imaging tools. Here, the present inventors propose the use of Manganese Enhanced Magnetic Resonance Imaging (MEMRI) as a new tool for evaluating *in vivo* the effects of modulators of astrocyte connexins, especially connexin 30 (Cx30) and connexin 43 (Cx43) inhibitors. They also propose to combine these modulators to manganese-based compounds for inducing antitumor and radiosensitizing effects.

**Background of the invention**

Glia is mostly composed of microglial cells, oligodendrocytes and astrocytes. Astrocytes are organized in plastic and tightly regulated networks, encoded by connexins (Cx) and especially connexines 30 (Cx30) and connexins 43 (Cx43), transmembrane proteins involved in gap junctions.

It is now widely accepted that astrocytes are connected through connexin-based gap junction channels (GJ), with brain region specificities. These networks modulate neuronal activities, such as those involved in sleep-wake cycle, cognitive, or sensory functions. Additionally, astrocyte domains have been involved in neurogenesis and neuronal differentiation during development. They participate in the "tripartite synapse" with both pre-synaptic and post-synaptic neurons by tuning down or up neuronal activities through the control of neuronal synaptic strength. In physiological situations, neuroglial networking is consequently resulting from a two-way interaction between astrocyte gap junction-mediated networks and those neurons. Connexins have been repeatedly involved in neurological and neurodegenerative disorders (cancers, cardiovascular diseases, wounds, pain, migraine, epilepsy, neurological conditions, neurodegenerative diseases, ischemia, eye disorders, hearing disorders, drug-induced liver injury, infectious diseases, cytotoxicity induced by chemotherapeutic agents, inflammatory disorders).

Over the past 10 years, knowledge about neuroglial interactions has widened and now includes effects of CNS-targeting drugs such as antidepressants, antipsychotics, psychostimulants, anti-epileptics, or sedative drugs as potential modulators of astrocyte networking activity.

Drugs used to treat neurological and psychiatric disorders have been generally studied through their neuronal effects. Yet, it is now clear that astrocytes are also targeted by those drugs, especially through the modulation of Cx30 and Cx43 levels of expression and function. Those modulations are either neuron-dependent or not, and might be linked to the aforementioned reciprocal interactions between neurons and astrocyte networks (Charveriat M. et al., 2017).

A number of studies have pointed out how tricyclic antidepressants such as clomipramine and imipramine modulate astrocyte Cx expression or functions. Of particular interest is the finding that Cx expression in astrocytes is modulated in animal models of depression as well as in depressed patients. Those data suggest a central role of astroglial Cx as modulators of the pharmacological profile of antidepressants (Charveriat M. et al., 2017).

The analysis of others classes of antidepressants such as SNRI (Serotonin-Norepinephrine Reuptake Inhibitor), NRI (Norepinephrine Reuptake Inhibitor) and SSRI (Selective Serotonin Reuptake Inhibitor) has shown contrasting effects after treatment of astrocyte cultures. Interesting data have also been gathered from antidepressant-treated rodents: chronic treatment with fluoxetine (SSRI) increased Cx43 expression in rat prefrontal cortex. Consistently, it has been shown that stressed rats presented a lower level of Cx43 expression and function in the cortex, while fluoxetine reversed those reductions. Alternatively, it has been proposed that antidepressants such as fluoxetine may exert their therapeutic activity by modulating the expression and/or activity of Cx43 in astrocytes. Those results emphasize the complex effects of different therapeutic classes of antidepressants on astroglial Cx, with various outcomes on expression and GJ and hemichannel functions as well as regional properties (Charveriat M. et al., 2017).

Psychiatric disorders such as schizophrenia, bipolar disorders, or autism are characterized by marked signs of glial dysfunction. More precisely, in autistic patients astroglial Cx43 expression is increased in superior frontal cortex. Antipsychotic drugs have been developed since the 1950s to reduce the symptoms of those disorders, and among them haloperidol, lithium, clozapine, or chlorpromazine. Those drugs typically modulate the expression and function of astrocyte Cx. Astrocyte Cx are also involved in the pathophysiology of epilepsy, as they are often disregulated in this pathology (Charveriat M. et al., 2017).

The effects of numerous anesthetics on astroglial Cx have been described for more than 20 years, notably in primary cultured astrocytes from the striatum or the cortex or in astrocytes from cortical slices. Altogether, those data reinforce the role that astrocytes play as controllers of sleep rhythm and complex cognitive processes such as sleep, through the modulation of their network organization.

Thus, connexins (in particular the astrocytic Cx) are involved in the pathophysiology of many psychiatric diseases. Yet, the results gathered so far are often contradictory, with various outcomes on the expression of the Cx, that may be due to regional properties and developmentally-modulated expression. For example, in astrocytes, Cx43 is distributed in a plaque-like manner between cells and is also located elsewhere in these cells compatible with cytoplasmic pools or hemichannels. This Cx appears post-natally while Cx30 is expressed several weeks after birth. In primary cultures, astrocytes express only Cx43 while Cx30 starts to be detected after 10 weeks, however, the addition of neurons induces expression of Cx30 in 3 week-old primary cultures. In general, Cx43 is predominant, but its expression varies by brain region and stage of development. For instance, hypothalamic astrocytes express four times more Cx43 than striatal astrocytes, and are more highly coupled. White matter astrocytes express minimal or no Cx30. In contrast, in the thalamus Cx30 is more highly expressed than Cx43 (Charveriat M. et al., 2017).

So far, the function and localization of these proteins have been studied mainly by using primary cultured cells or by staining the protein with antibodies on slices (by immunohistochemistry). These studies cannot detect real-time modulation and regionalization specificities of the connexin-expressing cells. Moreover, they cannot assess the *in vivo* biological effect of modulatory compounds on these cells.

There is therefore a need of more sensitive technics for studying *in vivo* the role of the connexins (for instance astrocytic connexins such as Cx43 and Cx30), and the biological effect of modulatory compounds on these particular proteins, in whole brain global studies.

Recent *in vivo* studies used imaging markers such as PET probes, passive fluorescent dyes or genetically encoded $Ca^{2+}$ indicators (GECIs). In particular, two-photon laser scanning microscopy (2PLSM) and laser scanning confocal microscopy (LSCM) have been used to study the functional relationships between astrocytes and neurons. However, the application of these probes has been limited due to local application, lack of specificity and low penetrance depth of optical microscopy.

In fact, studying astrocytes in their natural environment remains challenging because: (i) astrocytes are electrically silent; (ii) astrocytes and neurons express an overlapping repertoire of transmembrane receptors; (iii) the size of astrocyte processes in contact with synapses is below the resolution of confocal and two-photon microscopes, and (iv) bulk-loading techniques using fluorescent $Ca^{2+}$ indicators lack cellular specificity (Li et al, 2013).

More sensitive technics for whole brain imaging are therefore needed for analyzing, in a dynamic manner, the *in vivo* behavior of the astrocytes, their functional relationships with neurons and the effects of modulatory compounds on these particular cells.

The present invention fulfills this need, by providing a new tool to detect *in vivo* and in real-time the astrocytic influence in the brain and the role of these particular cells in the glial network.

The present invention also provides a new tool to detect in real-time the influence of connexin modulation in the brain and the role of these particular proteins in the glial network, as wells as the local regions affected by their modulation.

Finally, the present invention provides new tumor radiosensitizing combination products that help increasing the local cellular concentration of manganese-based compounds in order to favor the apoptotic effect of ionizing radiations (IR) or chemotherapeutic treatments.

## Description of the invention

The methods of the invention exploit the capabilities of manganese-enhanced magnetic resonance imaging (MEMRI) which provides viability-specific biological contrast agent through the accumulation of manganese ($Mn^{2+}$) or of derivatives thereof.

It is known that cerebral manganese can enter both neurons and astrocytes. That's why MEMRI has already been used to evaluate cerebral activation (i.e., neuronal and astrocytic activation), notably during neuro-inflammatory processes (Malheiros et al, 2015). Yet, these studies do not allow to distinguish the effect of neurons from the one of the astrocytic network. Post-study analysis by immunohistochemistry on fixed samples (e.g., with GFAP antibodies) was required to highlight the presence of activated astrocytes in the studied regions of the brain.

The present inventors show here for the first time that manganese accumulates in brain regions where connexins are inhibited by well-known connexin blockers (mefloquine, flecainide, and meclofenamic acid). They showed that, after administration of these connexin-blocking agents, accumulation of manganese generates a high MEMRI signal in different brain regions, depending on the used Cx-blocking agent. More precisely, the three tested connexins blockers, used at identical doses, increased manganese concentration, notably in hippocampus, striatum or somato-sensory cortex, in wild-type mice. Conversely, they differentially affected the MEMRI signal in dorsal raphe, hypothalamus, locus coeruleus, lateral parabrachial nucleus, olfactory bulb and substantia nigra. Such effects may result from different distribution or functions of Cx in each brain area. They are also linked to different pharmacodynamic profiles on neuroglial interaction (Jeanson et al, 2016; Duchêne et al, 2016; Charveriat et al, 2017).

Taken together, these findings demonstrate that MEMRI can be used for exploring the mechanism of action of connexin modulators as well as for identifying their specific target area.

In a first aspect, the present invention therefore relates to the use of the MEMRI imaging technology to study *in vivo,* in real time, the impact on the brain of a tested subject of candidate molecules that have or are thought to have a connexin-modulating activity.

As used herein, a "connexin-modulating activity" is an inhibiting or an activating effect on a connexin membrane protein or on its activity, directly or indirectly, said connexin being either involved in intercellular gap junctions, or in hemichannels regulating the intra-extra cellular communication. This effect may be reversible or not.

In other terms, the present invention relates to a screening method to study the impact on the brain of a tested subject of candidate molecules that have or are thought to have connexin modulating activity, comprising the following steps:

i) analyzing the MEMRI signal of at least one region of the brain of the tested subject in the absence of the candidate molecule,

ii) analyzing the MEMRI signal of at least one region of the brain of the tested subject in the presence of the candidate molecule,

iii) comparing the MEMRI signals obtained in steps i) and ii).

In this screening method, it is also possible to use several subjects, and to apply steps i) and ii) to different subjects. It is noteworthy that these two steps can be performed in any order.

The invention furthermore encompasses a method for analyzing a region of the brain of a tested subject, said method comprising performing manganese enhanced MRI (MEMRI) on the brain of said subject, prior and after the administration of at least one candidate molecule that has or is thought to have connexin modulating activity.

In these methods, the connexin modulating activity of said candidate molecule will be confirmed if and where the MEMRI signal is modified after said molecule is administrated (i.e., if and where $Mn^{2+}$ accumulation changes).

Said connexin-modulating activity is preferably a connexin-blocking agent, more preferably a glial connexin-blocking agent, even more preferably a connexin43 or a connexin30-blocking agent.

If the MEMRI signal is enhanced in at least one region of the brain after said molecule is administrated (i.e., if $Mn^{2+}$ accumulation enhances in at least one region of the brain), it is possible to detect in which region of the brain said connexin-blocking agent is specific and effective.

In the context of the invention, the MEMRI technology is preferably used for monitoring a therapeutic treatment implying the administration of a connexin-modulating agent (preferably a connexin-blocking agent) to a subject in need thereof. This subject is for example suffering from cancer, a cardiovascular disease, wound, pain, migraine, epilepsy, a neurological condition, a neurodegenerative disease, ischemia, eye disorders, hearing disorders, drug-induced liver injury, an infectious disease, cytotoxicity induced by chemotherapeutic agent, an inflammatory disorder. Said connexin-modulating agent is as defined below.

In addition, the MEMRI technology can be used for diagnosing diseases implying connexin deregulation. Said diseases are cancers, cardiovascular diseases, wounds, pain, migraine, epilepsy, neurological conditions, neurodegenerative diseases, ischemia, eye disorders, hearing disorders, drug-induced liver injury, infectious diseases, cytotoxicity induced by chemotherapeutic agents, inflammatory disorders.

The MEMRI technology is a non-invasive tool that is now well-known in the art and widely used for providing anatomical information of biological systems. It is now well described in the art. Conditions and principles are for example detailed in Massaad and Pautler, 2011.

It is known that despite the essential character of the manganese ion for the cellular function, overexposure of $Mn^{2+}$ can lead to neurotoxicity causing symptoms of bradykinesia or cognitive deficits in human (Crossgrove and Zheng, 2004; Aschner et al., 2007) and basal ganglia dysfunction in rodents (Bouabid et al., 2014). Although one of the possible approaches to minimize $Mn^{2+}$ toxicity is to administer $MnCl_2$ directly to the target region of interest (e.g., a specific brain region), it can also be injected systemically into the bloodstream or in the cerebrospinal fluid (Malheiros et al, 2015). In a preferred embodiment, in the methods of the invention, $Mn^{2+}$ is injected systemically to the target subject, intravenously, intraperitoneally, or subcutaneously. This systemic injection is indeed harmless for the tested subject. It is also possible to administer $Mn^{2+}$ intranasally (Massaad CA et al, 2011).

The methods of the invention can use any particular settings already disclosed in the art, especially those that avoid toxicity to occur (some of them are explained in the review of Malheiros et al, 2015 or in the cited studies). For example, it would be possible to use the following compounds, that have already been proposed and studied in the art: manganese chloride tetrahydrate (Lumenhance), Mn-(PyC3A) (Gale et al, 2015), CMC-001 (Jorgensen JT et al, 2012), EVP 1001-1 (SeeMore™), calmangafodipir ($Ca_4Mn(DPDP)_5$ or PledOx), mangafodipir (MnDPDP) and/or MnPLED (Karlsson JOG 2015).

Preferably, the manganese-based compounds used in the invention have a molecular weight which is lower than 1kDa, in order to be able to enter the cells via Gap Junctions (GJ) composed of connexins.

In a preferred embodiment, the MEMRI technology used in the present invention requires the administration of the FDA-approved mangafodipir, a $Mn^{2+}$ chelate with the ligand fodipir.

Other Mn-based compounds that can be used as MRI contrast agent in the context of the invention are disclosed in the following table:

| Mn-based compound | Bibliographic References |
|---|---|
| *Polycarboxylic acid ligands* | |
| Mn-EDTA/EDTA-DPP | Unger E, et al. 1993 |
| Mn-EDTA-BOM | Aime S, et al.,. 2002 |
| Mn-EDTA-diphenylcyclohexyl | Troughton JS, et al. 2004 |
| Mn-DTPA-SA | Schwendener RA, et al., 1990 |
| Mn-TTHA | Rongved P, Klaveness J. 1991 |
| *Porphyrins (sulfonatoporphyrins)* | |
| Mn-PcS4 Mn-TPPS4 Mn-TPPS3 Mn-TPPS2 | Place DA, et al, 1992 |
| Mn-Mesoporphyrin | Schmiedl UP, et al, 1993 |
| Mn-Hematoporphyrin | Fawwaz RA, et al, 1971 |
| UROP-1 | Schmiedl UP, et al, 1993 |
| ATM-10 | Fujimori H, et al. 1997 |
| Mn-TPP | Ni Y et al, 1997 |
| HOP-8P | Takehara Y, et al., 2002 |
| Gd-ATN-10 | Matsumura A, 1997 |
| *Inorganic particle/bulk materials* | |
| MnO, MnO2, Mn3O4, MnCO3 | Shapiro EM, et al. 2008 |
| *Mn-doped iron oxide nanoparticle* | |
| Mn-Fe oxide composite of rod, wire and needle shape (400-1000 nm) | Leung KC, et al, 2009 |
| Mn-doped SPIO coated with mPEG-*b*-PCL | Lu J, et al. 2009 |
| Multifunctional magneto-polymeric nanohybrids (MMPNs) | Yang J, et al. 2007 |
| *Manganese clusters* | |
| 'Single molecule magnet' [Mn12O12(O2CCH3)16(H2O)4] | Mertzman JE, et al. 2009 |
| MnPOMs: MnSiW11 and MnP2W17 | Li Z, et al. 2007 |
| *Manganese organic frames* | |
| Mn-NMOFs | Taylor KM, et al 2008 |
| *MnO-based nanoparticle approach* | |
| PEG-coated MnO | Na HB, et al 2007. |
| HMONs and WMONs | Shin J, et al 2009 |
| Phospholipid-coated MnO | Pan D, et al 2009 |
| *Polymeric and 'Soft' particle approach* | |
| Manganese oleate nanocolloids | Pan D, et al 2009 |
| 'NanoBialys' [Mn(III)-loaded polymeric agent] | Pan D, et al 2008 |

(continued)

| Polymeric and 'Soft' particle approach | |
| --- | --- |
| Dendritic manganese DTPA | Steibel BA, et al 2009 |

In the context of the invention, the "tested subject" can be any laboratory animals, for example rats (Aoki et al., 2004), mice (Watanabe et al., 2002), rabbits (Zhao et al., 2015), fish (Scadeng M., 2009) and so on. It can also be a human being. Said animal or human can be healthy or suffer from a disease, such as a neurodegenerative disease or a neurological or psychiatric disease. Said neurodegenerative disorder is for example Alzheimer's disease, corticobasal degeneration, Creutzfeldt-Jacob disease, frontotemporal lobar degeneration, Parkinson's disease, Huntington's disease, Tourette's syndrome, normal pressure hydrocephalus, organic chronic brain syndrome, Pick disease, progressive supranuclear palsy, senile dementia, vascular dementia, Amyotrophic Lateral Sclerosis, or multiple sclerosis. Said neurological disorder is for example epilepsy, chronic or acute pain, neuropathic pain, sleep disorders, obstructive sleep apneas, attention deficit - hyperactivity disorder. Said psychiatric disorder is for example a mood disorder, an anxiety disorder, schizophrenia, tinnitus, depression, dementia, manic episode, obsessive-compulsive disorder, senility, dependence, an addiction or a bipolar disorder, fibromyalgia. The tested subject can also suffer from a neurological disease, an eye disorder, an hearing disorder, an oculodentodigital dysplasia (ODDD) or a glioblastoma.

In a preferred embodiment, the tested subject is a non-human laboratory animal.

By applying the methods of the invention on healthy animal models, it is possible to analyze in real-time the structural and functional relationships between astrocytes and neurons in intact or perturbed conditions. This research tool may be used by all the scientists studying the astrocyte network, astrocyte-neuron crosstalk, and the modulatory activity of connexin-blocking agents. It is also possible to highlight the function and localization of connexin-expressing cells *in vivo.* In this case, the candidate molecule used in the method of the invention is typically a connexin-blocking agent. This application requires the same steps as described above, which are to analyze the MEMRI signal of at least one region of the brain of a tested subject in the absence and in the presence of a connexin-blocking agent, and compare the strength and the localization of the signals in both cases. In this case, the tested subjects are preferably healthy laboratory animals.

By applying the methods of the invention on pathologic animal models (naturally sick or artificially obtained) and comparing them with healthy control subjects, it is possible to see if connexin-expressing cells and/or connexins are affected or modulated in a pathogenesis or its symptoms. More generally, by comparing the MEMRI signals observed in pathologic versus healthy subjects, one can conclude if and where said pathology involves an over-expression of connexins if and where the MEMRI signal resumes to its normal level after a connexin-inhibiting compound is administered, or if the MEMRI signal differs in pathologic versus healthy subjects before the connexin-inhibiting compound is administered. Additionally, one can conclude if and where said pathology involves an under-expression of connexins if and where the MEMRI signal is not affected when a connexin-inhibiting compound is administered. Accordingly, it will be possible to select appropriate drugs to target the connexins in order to alleviate the symptoms of the disease, when connexins are involved in same.

In the context of the invention, the "candidate molecules" can be any molecule that may have an impact on connexins. It can be a chemical molecule, a protein, a protein fragment or a nucleic acid (RNAi) capable of modulating the expression and/or functional activity of connexins, directly and/or indirectly. Preferably, said molecule is thought to have an inhibitory activity on the connexins. Said molecule can be for example a psychotropic agent, an anti-inflammatory agent (such as MFA), or any other molecule acting on connexins (flecainide, Mefloquine, etc.). Preferably, said candidate molecule is able to cross the brain-blood barrier.

In the context of this invention, a "connexin-blocking" agent is a chemical molecule, a protein, a protein fragment or a nucleic acid (for example RNAi) capable of inhibiting the expression and/or the functional activity of connexins, directly and/or indirectly. In the context of this invention, the connexin-blocking agents are advantageously chosen from: flecainide (preferably the R enantiomer or a racemic mixture containing same), long-chain alcohols (for example, heptanol and octanol), fenamates (for example, meclofenamic acid, mefenamic acid, flufenamic acid, niflumic acid, tolfenamic acid), arylaminobenzoates, aminosulfonates (for example taurine), glycyrrhetinic acid derivatives (for example, $18\text{-}\beta$-glycyrrhetinic acid, $18\text{-}\alpha$-glycyrrhetinic acid and carbenoxolone), oleamides (for example, cis-9-octadecenamide), or tetraalkylammonium ions and polyamines (such as spermine and spermidine), quinine derivatives (such as mefloquine, quinine, quinidine), 2-ABP, anesthetic agents (halothane, enflurane or isoflurane), cyclodextrins ($\alpha$-cyclodextrin ($\alpha$-CD), $\beta$-cyclodextrin ($\beta$-CD), and $\gamma$-cyclodextrin ($\gamma$-CD)), antibodies directed against the extracellular domain of the connexins or peptides with conserved patterns mimicking this particular domain (in particular Gap26, Gap27, peptide 5, Gap19, L2, JM2, CT9, AAP10), oleic acid, palmitoleic acid, decaenoic acid, myristoleic acid, staurosporine, strophanthidin, ouabain, delta-9-tetrahydrocannabinol, 2-aminoethoxydiphenyl borate acetic acid, propionic acid, 12-O-tetradecanoylphorbol-13-acetate (TPA), 2,3 butandione monoxime, carbachol, noradrenaline, FGF-2, angiotensin-II, Atrial Natriuretic factor ANF, VEGF, 1-oleyl-2-acetyl-*sn*-glycerol, 11,12-epoxyeicosatrienoic acid, lidocaine, tonabersat (SB-220453, (cis-(-)-6-acetyl-4S-(3-chloro-4-fluorobenzoylamino) -3,4-dihydro-2,2-dimethyl-2H-1-benzopyran-3S-ol), Nexagon and Peptagon (from CoDa Therapeutics). These different molecules are specifically described in the following articles: Srivinas M, 2009; Srinivas M, 2003; Harks EG, 2001; and Salameh A, 2005; Dhein S., 2004; Duchêne et al, 2016; and Leybaert L et al, 2017).

Another application of the methods of the invention is to identify new treatments associating connexin-modulators and psychotropic drugs. As a matter of fact, it is known that targeting connexins can result in the change of properties of CNS drugs, in terms of pharmacological profiles (WO 2010/029131, WO 2013/064579, WO 2015/011246). The MEMRI technology may help determine the mechanism of action of these combinations, notably in terms of localization of the connexin modulation in brain. The selection of appropriate combination products (connexin-blocking agents and psychotropic drug) will be eased. In this case, the connexin-modulators and tested psychotropic drugs can be administered separately or concomitantly, and the MEMRI signal analyzed before and after said administration(s). Normal and pathologic animal models can be used.

Last but not least, the present inventors propose to use the MEMRI imaging method exposed above to study the astrocyte-neuron networks *in vivo.*

The coupling between astrocytes has been shown to be significantly reduced in cells lacking Cx43, notably in hippocampus, demonstrating that this isoform is a major support for direct intercellular communication in astrocytes (Rouach N et al, 2008). Moreover, in the brain, Cx43 is mostly expressed by astrocytes (Charveriat et al, 2017). The administration of a specific Cx43-inhibiting compound will affect astrocytic cells in the brain, where, as exposed above, modulation of $Mn^{2+}$ concentration and thus a strong modification of MEMRI signal is likely to be observed.

Therefore, by using specific Cx43-inhibiting compounds, one can observe the behavior of the astrocytic cell connexins of the brain in real-time, *in vivo,* in a selective manner.

In another aspect, the present invention therefore relates to the use of the method of the invention to study the astrocyte-neuron networks in the brain of a tested subject. In this case, a connexin-modulating compound, preferably a connexin-blocking agent as defined above, is administered. More preferably, a specific Cx43-blocking agent or a specific Cx30-blocking agent is administered to said tested subject.

Said Cx43-blocking compound is for example Mefloquine. Mefloquine inhibits the different functions coded by Cx43, gap junctions and hemichannels, most likely via the promotion of channel conductance closure (Picoli C et al, J. Biomol. Screen 2012; Jeanson T et al, Scientific reports 2016).

Said Cx43-blocking compound can also be a siRNA or a shRNA inhibiting the Cx43 expression. Said antisense RNA can be for example carried by a lentivirus, whose envelope is known to have a specific tropism towards astrocytes.

It is also possible to use a compound that inhibits both Cx30 and Cx43 which are both known to be expressed mainly by astrocytic cells in the brain. Flecainide and meclofenamic acid can be used in this respect (Duchêne A. et al, 2016). Also, it is possible to use siRNAs or shRNAs inhibiting both the Cx30 and the Cx43 expression. Said anti-sense RNAs can be for example carried by a lentivirus, whose envelope is known to have a specific tropism towards astrocytes.

Preferably, said connexin-blocking agents are able to cross the brain-blood barrier.

In this particular application, the tested subject may be a healthy or a pathogenic non-human laboratory animal model. With the use of mangafodipir or other non-toxic $Mn^{2+}$ analogs, it can also be a human being, healthy or not.

Pathogenic animal models or unhealthy human beings are for example suffering from any neurological, neurodegenerative or psychiatric disorders mentioned above. The tested subject can also suffer from an eye disorder, a hearing disorder, an oculodentodigital dysplasia (ODDD) or a glioblastoma.

The localization of Cx30- and/or Cx43-expressing cells (and their behavior once Cx43 and/or Cx30 are inhibited) using MEMRI might also be interesting to analyze the astrocytic network in the brain of subjects suffering from these disorders.

The MEMRI technology used in this second aspect can rely on the administration of any of the above-mentioned manganese-based compound.

Preferably, it has a molecular weight lower than 1kDa.

Altogether, the present inventors propose a new tool to provide insights about neuron-astrocyte interaction in clinical trials and to pharmacologically evaluate new modulators of those interactions.

Based on their observation that manganese accumulates in cells upon connexin inhibition, the present inventors finally propose to combine said connexin-blocking agents to manganese-based compounds for inducing antitumor, radiosensitizing or chemosensitizing effects.

By blocking connexins and especially the connexin 43, it will be possible to induce the accumulation in specific cells of the brain (e.g., astrocytes) of manganese-based compounds that can sensitize the cells to Ionizing Radiations (IR) or chemotherapeutic treatments. Consequently, it will be possible to lower the doses of IR or chemotherapy, and/or enhance IR or chemotherapy anti-tumor efficiency, especially in patients suffering from cancers such as glioblastomas and astrocytomas.

In another aspect, the present invention therefore relates to a combination product containing a connexin-blocking agent and a manganese-based compound for simultaneous, separated, or staggered use for treating patients suffering from cancer, by enhancing IR anti-tumor efficiency in same or by enhancing the anti-tumor effect of the manganese-based compound in same.

Several manganese-based compound have been proposed to radio sensitize tumor cells:

- A manganese porphyrin, MnHex-2-PyP, has been shown to substantially reduce cell viability, clonogenic cell survival, and DNA damage repair in tumor cells having undergone IR. It exerts a radiosensitizing effect in tumor models both in vitro and in vivo via pro-oxidative actions and therefore bears a large therapeutic potential (Shin SW et al., 2017).

- Boronated metalloporphyrins such as MnBOPP (manganese chelate of 2,4-(alpha, beta-dihydroxyethyl) deuterioporphyrin IX) has been shown to accumulate in glioma and can be used for neutron capture therapy (Huang LR et al, 1993).

Said patients are for example suffering from diffuse astrocytic and oligodendroglial tumors, other astrocytic tumors, ependymal tumors, other gliomas, choroid plexus tumors, neuronal and mixed neuronal-glial tumors, tumors of the pineal region, embryonal brain tumors, tumors of cranial and paraspinal nerves, meningiomas, mesenchymal brain tumors, non-meningothelial tumors, melanocytic tumors, cerebral lymphomas, histiocytic tumors, germ cell tumors, tumors of the sellar region, metastatic brain tumors or other types of brain tumors, as classified by WHO in 2016 (Louis DN et al, 2016).

Other manganese-based compounds have been proposed to enhance the efficiency of chemotherapeutic treatments as well: specifically, MnTnBuOE-2-PyP caused a significant decrease in tumor clonogenicity in combination with irradiation, and also in combination with 5-fluorouracil with and without Mitomycin C. Kosmacek EA et al. demonstrated that MnTnBuOE-2-PyP could be used for enhancing radiation and chemotherapy treatment for rectal and anal cancers (Kosmacek EA et al., 2016).

The combination product of the invention, when containing such manganese-based compound, could therefore be used for enhancing the chemotherapeutic effect of conventional chemotherapies, as well as IR, in patients in need thereof.

Said "conventional chemotherapies" are for example Aflibercept, AMG-102, AP12009, ATI3387, Bevacizumab, BIBW2992, Bortezomib, Carboplatin, Carmustine, Cediranib, Cetuximab, Cilengitide, CT-322, Dasatinib, Deforolimus, Depsipeptide, Enzastaurin, Erlotinib, Erlotinib + Temsirolimus, Erlotinib Or Tipifarnib Or Pazopanib + Lapatinib, Etoposide, Everolimus, Gefitinib, Gefitinib + Everolimus, Gefitinib + Sirolimus, Imatinib, Irinotecan, Lapatinib, LBH589, Lomustine, Lonafarnib, Nimotuzumab, Nintedanib, Nitrosoureas, Pazopanib, Perifosine, Procarbazine, Ramucirumab, Sirolimus, Sorafenib, Sorafenib + Temsirolimus, Sunitinib, Tandutinib, Temozolomide, Temsirolimus, Thalidomide, Tipifarnib, Vandetinib, Vatalanib, Vincristine, Vorinostat, XL184, XL765 (Berghoff AS, 2018; Wen PY, 2008).

In this combination product, the manganese-based compound is chosen in the group consisting of: manganese porphyrin, boronated manganese metalloporphyrin, or MnTnBuOE-2-PyP. It can also be any compound containing manganese that is known to be radio or chemotherapeutically sensitizing and preferably has a molecular weight lower than 1kDa.

In this combination product, the connexin-blocking agent is preferentially selected from: flecainide (preferably the R enantiomer or a racemic mixture containing same), long-chain alcohols (for example, heptanol and octanol), fenamates (for example, meclofenamic acid, mefenamic acid, flufenamic acid, niflumic acid, tolfenamic acid), arylaminobenzoates, aminosulfonates (for example taurine), glycyrrhetinic acid derivatives (for example, 18-β-glycyrrhetinic acid, 18-α-glycyrrhetinic acid and carbenoxolone), oleamides (for example, cis-9-octadecenamide), or tetraalkylammonium ions and polyamines (such as spermine and spermidine), quinine derivatives (such as mefloquine, quinine, quinidine), 2-ABP, anesthetic agents (halothane, enflurane or isoflurane), cyclodextrins (α-cyclodextrin (α-CD), β-cyclodextrin (β-CD), and γ-cyclodextrin (γ-CD)), antibodies directed against the extracellular domain of the connexins or peptides with conserved patterns mimicking this particular domain (in particular Gap26, Gap27, peptide 5, Gap19, L2, JM2, CT9, AAP10), oleic acid, palmitoleic acid, decaenoic acid, myristoleic acid, staurosporine, strophanthidin, ouabin, delta-9-tetrahydrocannabinol, 2-aminoethoxydiphenyl borate acetic acid, propionic acid, 12-O-tetradecanoylphorbol-13-acetate (TPA), 2,3 butandione monoxime, carbachol, noradrenaline, FGF-2, angiotensin-II, Atrial Natriuretic factor ANF, VEGF, 1-oleyl-2-acetyl-sn-glycerol, 11,12-epoxyeicosatrienoic acid, lidocaine, tonabersat (SB-220453, (cis-(-)-6-acetyl-4S-(3-chloro-4-fluorobenzoylamino)-3,4-dihydro-2,2-dimethyl-2H-1-benzopyran-3S-ol), Nexagon and Peptagon (from CoDa Therapeutics). Antisense RNAs are also encompassed.

Anti-sense RNAs, Mefloquine, Flecainide and MFA are preferably used in the combination product of the invention.

In another preferred embodiment, the present invention specifically pertains to the use of a connexin-blocking agent and a manganese-based compound for treating patients suffering from cancer, by enhancing IR anti-tumor efficiency in same or by enhancing the anti-tumor effect of the manganese-based compound in same.

In a particular embodiment, the present invention also relates to a therapeutic composition comprising a connexin-blocking agent and a manganese-based compound, which can advantageously be used for treating patients suffering from cancer by enhancing IR anti-tumor efficiency in same or by enhancing the anti-tumor effect of the manganese-based compound in same.

The present invention moreover relates specifically to the use of a connexin-blocking agent and a manganese-based compound in the preparation of a medicament that is intended to be used for treating patients suffering from cancer, preferably glioblastoma or astrocytoma.

This composition is preferably formulated for patients suffering from cancer. In addition to said connexin-blocking agent and to said manganese-based compound, the composition can comprise any pharmaceutical vehicle, stabilizer, adjuvant and the like as frequently used in the art.

Examples of pharmaceutically acceptable vehicles include, but are not limited to: water; aqueous vehicles such as, but not limited to, sodium chloride solution, Ringer's solution, dextrose solution, dextrose and sodium chloride solution, and lactated Ringer's solution; water-miscible vehicles such as, but not limited to, ethyl alcohol, polyethylene glycol, and polypropylene glycol; and nonaqueous vehicles such as, but not limited to, corn oil, cottonseed oil, peanut oil, sesame oil, ethyl oleate, isopropyl myristate, and benzyl benzoate.

According to a preferred embodiment, this composition is formulated for oral administration (including buccal cavity or sublingually). Other interesting formulations include formulations for intraperitoneal (i.p.), intravenous (i.v.), subcutaneous (s.c.), intramuscular (i.m.), transcutaneous, transdermal, intrathecal and intracranial administrations. Still other formulations include epidural, submucosal, intranasal, ocular cul-de-sac and rectal routes of administration, as well as administration by pulmonary inhalation.

A variety of administration means, including but not limited to capsules, tablets, syrups, creams and ointments, suppositories, patches or any reservoir capable of containing and dispensing the active principles mentioned above, can be used for formulating the above-described compositions.

In a final aspect, the present invention relates to an *in vitro* method of identifying a combination product that may potentiate a radiotherapy or a chemotherapeutic treatment in a subject, said method comprising the steps of:

(a) adding a candidate connexin-blocking agent to a tumor cell culture,

(b) adding a manganese-based candidate compound to said culture, optionally washing the cells by conventional means,

(c) measuring the intracellular concentration of said manganese-based candidate in the tumor cell culture, e.g. by MRI, mass spectrometry, spectrophotometry or using chemical reactions or physical methods, and

(d) measuring the intracellular concentration of said manganese-based candidate in a tumor cell culture in the absence of said candidate connexin-blocking agent, e.g. by MRI,

wherein said manganese-based candidate and said candidate connexin-blocking agent are selected in said combination product if the intracellular concentration of step c) is at least two times, preferably three times, more preferably four times higher than the concentration of step d).

Said tumor cells are for example cell lineages or primary cells of astrocytic and oligodendroglial tumors, ependymal tumors, gliomas, choroid plexus tumors, neuronal and mixed neuronal-glial tumors, tumors of the pineal region, tumors of cranial and paraspinal nerves, meningiomas, mesenchymal tumors, non-meningothelial tumors, melanocytic tumors, cerebral lymphomas, etc.

## Examples

### 1. Materials and Methods

### Animals

The MRI measurements were performed on 42 wild-type C57BL/6 male mice. All mice weighed between 25 and 35 g. Mice were housed 4-5 per cage under standard conditions (12/12 h light-dark cycle, light on at 7 am, 22 $\pm$ 1°C ambient temperature, 60% relative humidity). This work was carried out in accordance with the Policies of the French Committee of Ethics. Animal surgery and experimentations conducted in this study were authorized by the French Direction of Veterinary Services and all efforts were made to improve animal welfare and minimize animals suffering.

**Drugs and administration**

Mefloquine (MEF; Sigma-Aldrich), meclofenamic acid (MFA; Sigma-Aldrich), flecainide acetate salt (FLE; Sigma-Aldrich) or its vehicle (0.9% NaCl with 2% DMSO) were freshly prepared and administrated intraperitoneally at 1 mg/kg, 2.5h before imaging, at 5 mL/kg body weight.

**MRI Acquisitions**

All animals were injected intraperitoneally with $MnCl_2$ 50 mg/kg. Animals received an injection of treatment (or saline) 24h after the $MnCl_2$ administration, and were imaged 2.5h later.

The MR acquisitions were performed on horizontal bore small animal scanners (Bruker BioSpin, Ettlingen, Germany) operating at 7T and 11.7T using a 4-channel mouse brain array coil and a two-channel mouse brain cryoprobe, respectively. The mice were anesthetized with 0.5-1.0% isoflurane in air throughout the experiments. The respiration rate was monitored and the body temperature was maintained at 36.5 °C using an MR-compatible, feedback-controlled air heating system (model 1025; SA Instruments, NY). A capillary filled with 100 μM $MnCl_2$ was placed on the head, inside the RF coil, and was used as signal intensity reference.

The $T_1$-weighted images were acquired using a 3D rapid acquisition with relaxation enhancement (RARE) sequence: RARE factor 4, repetition time/echo time = 250 ms / 6 ms, 4 averages. For $T_1$ relaxation time measurements, a true fast Imaging with Steady-state free Precession (trueFISP) sequence was used with the following acquisition parameters: repetition time/echo time = 3.66/1.83 ms, flip angle = 10, inversion time = 80 ms, number of inversion times = 60, 2 averages.

The spatial resolutions and acquisition times for the two sequences on the two MRI systems are listed below:

| | Acquisition time (min) | | Spatial resolution | |
| --- | --- | --- | --- | --- |
| | 7T | 11.7T | 7T | 11.7T |
| RARE | 80 | 47 | 0.1 x 0.1 x 0.1 mm$^3$ | 0.1 x 0.1 x 0.1 mm$^3$ |
| FISP | 60 | 70 | 0.15 x 0.15 x 0.15 mm$^3$ | 0.1 x 0.1 x 0.1 mm$^3$ |

**Data Processing**

The position of the brain image was co-registered to a template using SPM8 software (Welcome Trust Center for Neuroimaging, UK). The template image was co-registered to mouse brain atlas (Paxinos and Franklin's the Mouse Brain in Stereotaxic Coordinates 3rd Edition). $T_1$-weighted signals were normalized using the signal intensity from the reference tube. $T_1$ maps were calculated as described previously elsewhere (74). Eighteen regions of interest (ROIs) were defined using the mouse brain atlas.

The averaged $T_1$-weighted signals and $T_1$ values were calculated using an in house written Matlab program. To compensate for differences in coil sensitivity between left and right channels we calculated the signal intensities in ROIs drawn in the left and right cerebral cortex, including the somatosensory cortex and insular cortex, ($ROI_{cortex\_left}$ $ROI_{cortex\_right}$). The averaged T1-weighted signal intensity on the left side ($SI_{left}$) was then corrected following the equation:

$$SI_{left\_corrected} = SI_{left} \times SI_{cortex\_right} / SI_{cortex\_leftt} \qquad (1)$$

**Statistics**

Statistical analyses were performed using GraphPad Prism version 7.0c. Data were analyzed using Mann-Whitney non-parametric test, paired *t* test or one sample *t* test compared to the hypothetical value 1 or 100 as appropriate. In each experiment, a level of $p < 0.05$ was accepted as evidence for a statistically significant effect. * $p < 0.05$; ** $p < 0.01$; *** $p < 0.001$. Response of the MEMRI signal after drugs administration are expressed as means $\pm$ SEM of percent of control. The remaining data are expressed as mean $\pm$ SEM. When analyzing the specific response of the MEMRI after drugs administration, we choose to select six brain areas on the basis of their different response patterns.

**2. Results**

The *in vivo* impact of pharmacological modulators of Cx43, mefloquine, flecainide and meclofenamic acid, was investigated using MEMRI. These connexin modulators significantly modified Mn concentration in the hippocampus. The results are provided on Table 1 (flecainide), Table 2 (MFA) and Table 3 (mefloquine) for each brain region.

For all, MEMRI signals are expressed as percentage of control. Statistical significances are based on the comparison to the hypothetical value 100%.

Legend of the Tables: ns = not significant. SC = Somatosensory cortex; Cpu = Caudate putamen; Dra = Dorsal raphe pallidus; Hip = Hippocampus; Hyp = Hypothalamus; LC = Locus coeruleus; LPB = Lateral parabrachial nucleus; MC = Motor cortex; OB = Olfactory bulb; SN = Substantia nigra; ThN = Thalamic nuclei; OC = Orbital cortex, PrL = Prelimbic cortex; AC = Auditory cortex; IC = Insular cortex; Cg = Cingulate cortex; mPFC = Medial prefrontal cortex; Sep = Septum.

**Table 1: Specific responses of the MEMRI signal on brain regions after flecainide administration.**

| Region | T1-w signal intensity (% of control) | Statistics |
|---|---|---|
| SC | $120 \pm 4.88$ | < 0.01 |
| Cpu | $120 \pm 6.01$ | < 0.05 |
| DRa | $122 \pm 6.86$ | < 0.05 |
| Hip | $118 \pm 4.73$ | < 0.05 |
| Hyp | $121 \pm 7.05$ | < 0.05 |
| LC | $119 \pm 5.69$ | < 0.05 |
| LPB | $119 \pm 6.07$ | < 0.05 |
| MC | $122 \pm 5.09$ | < 0.01 |
| OB | $123 \pm 9.21$ | ns |
| SN | $117 \pm 6.97$ | ns |
| ThN | $121 \pm 5.47$ | < 0.05 |
| OC | $123 \pm 8.23$ | < 0.05 |
| PrL | $121 \pm 7.09$ | < 0.05 |
| AC | $114 \pm 4.04$ | < 0.05 |
| IC | $118 \pm 5.62$ | < 0.05 |
| Cg | $123 \pm 5.73$ | < 0.05 |
| mPFC | $121 \pm 6.86$ | < 0.05 |
| Sep | $119 \pm 6.95$ | < 0.05 |

**Table 2: Specific responses of the MEMRI signal on brain regions after meclofenamic acid administration.**

| Region | T1-w signal intensity (% of control) | Statistics |
|---|---|---|
| SC | $120 \pm 4.90$ | < 0.01 |
| Cpu | $130 \pm 3.90$ | < 0.001 |
| DRa | $112 \pm 5.31$ | ns |
| Hip | $118 \pm 4.32$ | < 0.01 |
| Hyp | $118 \pm 5.38$ | < 0.05 |
| LC | $104 \pm 6.23$ | ns |
| LPB | $106 \pm 6.15$ | ns |
| MC | $122 \pm 4.61$ | < 0.01 |
| OB | $147 \pm 4.91$ | < 0.001 |
| SN | $118 \pm 5.67$ | < 0.05 |
| ThN | $120 \pm 4.87$ | < 0.01 |
| OC | $138 \pm 4.69$ | < 0.001 |
| PrL | $136 \pm 4.41$ | < 0.001 |
| AC | $117 \pm 5.68$ | < 0.05 |
| IC | $128 \pm 5.17$ | < 0.01 |
| Cg | $127 \pm 4.49$ | < 0.01 |
| mPFC | $136 \pm 4.33$ | < 0.001 |
| Sep | $136 \pm 3.68$ | < 0.001 |

**Table 3: Specific responses of the MEMRI signal on brain regions after mefloquine administration.**

| Region | T1-w signal intensity (% of control) | Statistics |
|---|---|---|
| SC | 116 ± 5.39 | < 0.05 |
| Cpu | 123 ± 5.94 | < 0.05 |
| DRa | 113 ± 6.84 | ns |
| Hip | 117 ± 5.95 | < 0.05 |
| Hyp | 113 ± 8.39 | ns |
| LC | 111 ± 7.61 | ns |
| LPB | 111 ± 6.96 | ns |
| MC | 118 ± 5.44 | < 0.05 |
| OB | 126 ± 9.23 | < 0.05 |
| SN | 119 ± 6.33 | < 0.05 |
| ThN | 119 ± 5.01 | < 0.05 |
| OC | 127 ± 6.54 | < 0.01 |
| PrL | 125 ± 6.78 | < 0.05 |
| AC | 118 ± 5.94 | < 0.05 |
| IC | 125 ± 5.29 | < 0.01 |
| Cg | 120 ± 5.94 | < 0.05 |
| mPFC | 125 ± 7.21 | < 0.05 |
| Sep | 125 ± 8.32 | < 0.05 |

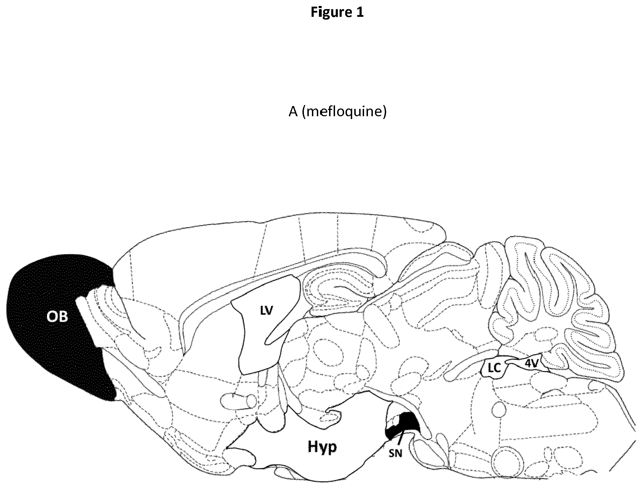

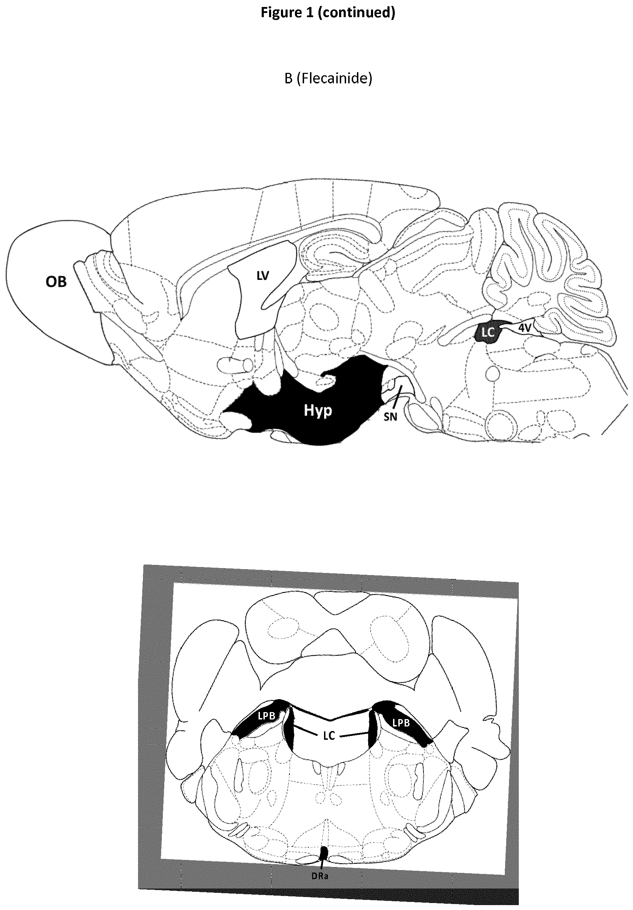

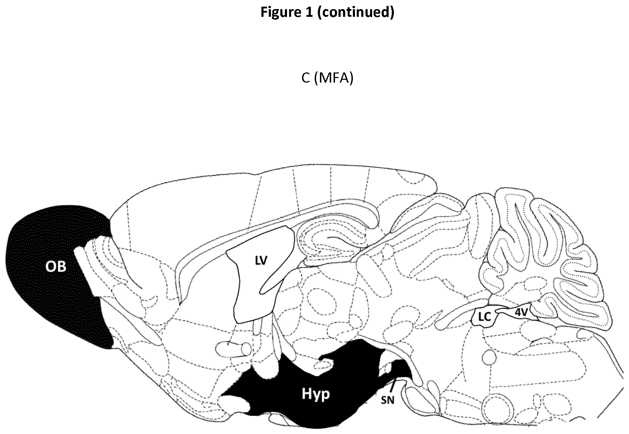

Six regions displaying different MEMRI response patterns to the three drugs were selected (Fig. 1 and Table 4).

**Table 4: Responses of the MEMRI signal on selected brain regions after mefloquine, flecainide and MFA administration (selected regions where those with different MEMRI signals between the three drugs).**

| Region | $T_1$-w signal intensity (% of control) | | | Statistics | | |
|---|---|---|---|---|---|---|
| | MEF | FLE | MFA | MEF | FLE | MFA |
| DRa | 113 ± 6.84 | 122 ± 6.86 | 112 ± 5.31 | ns | < 0.05 | ns |
| Hyp | 113 ± 8.39 | 121 ± 7.05 | 118 ± 5.38 | ns | < 0.05 | < 0.05 |
| LC | 111 ± 7.61 | 119 ± 5.69 | 104 ± 6.23 | ns | < 0.05 | ns |
| LPB | 111 ± 6.96 | 119 ± 6.07 | 106 ± 6,15 | ns | < 0.05 | ns |
| OB | 126 ± 9.23 | 123 ± 9.21 | 147 ± 4.91 | < 0.05 | ns | < 0.001 |
| SN | 119 ± 6.33 | 117 ± 6.97 | 118 ± 5.67 | < 0.05 | ns | < 0.05 |

Data from MEMRI imaging of treated groups were expressed as percentage of the control group and compared to the hypothetical value 1 (one sample t test).

It is demonstrated here that mefloquine administration significantly increases $T_1$-w signal intensity in the olfactory bulb and the substantia nigra (n= 6; +26.5% and +19,2%; both p<0,05; one sample t test; Fig. 1A *black region*), i.e. significantly increases manganese concentration in those regions.

In the same way, flecainide significantly improves this signal; approximately +21.9%; +20.9%; +19.2% and +19% respectively for the dorsal raphe pallidus, the hypothalamus, the locus coeruleus and the lateral parabrachial nucleus (n = 6; for each p<0,05; one sample t test; Fig. 1B black region),i.e. significantly increases local manganese concentration in those regions.

Finally, the meclofenamic acid is also able to significantly increase the $T_1$-w signal intensity by approximately +18.5%; +47.4% and +17.9% for the hypothalamus, the olfactory bulb and the substantia nigra regions, respectively (n=6; p<0,05 for the hypothalamus and the substantia nigra, p<0,001 for the olfactory bulb; one sample t test; Fig. 1C black region), i.e. significantly increases local manganese concentration in those regions.

Taken together, these findings demonstrate that MEMRI could be used for exploring the mechanism of action of connexin modulators as well as for identifying their specific target area.

Moreover, these findings demonstrate that MEMRI is useful tool for exploring the mechanism of connexin modulators as well as identify their specific target area in the brain. Additionally, these findings demonstrate that pharmacological modulation of cerebral connexins can increase local concentration of Mn-based compounds in different brain regions.

**Figure legend**

Figure 1 discloses a view of the regions that are differentially affected by treatments with Mefloquine (A), Flecainide (B), or MFA (C).

**Bibliographic references**

Aime S, Anelli PL, BOtta M, Brocchetta M, Canton S, Fedeli F, Gianolio E, Terreno E. Relaxometric evaluation of novel manganese (II) complexes for application as contrast agents in magnetic resonance imaging. J Biol Inorg Chem. 2002;7:58-67.

Aoki I, Naruse S, Tanaka C. Manganese-enhanced magnetic resonance imaging (MEMRI) of brain activity and applications to early detection of brain ischemia. NMR Biomed. 2004 Dec;17(8):569-80. Aschner M, Guilarte TR, Schneider JS, Zheng W. Manganese: recent advances in understanding its transport and neurotoxicity. Toxicol Appl Pharmacol. 2007 Jun 1;221(2):131-47.

Berghoff AS, Preusser M. Anti-angiogenic therapies in brain metastases. Memo. 2018;11(1):14-17 Bouabid S, Delaville C, De Deurwaerdère P, Lakhdar-Ghazal N, Benazzouz A. Manganese-induced atypical parkinsonism is associated with altered Basal Ganglia activity and changes in tissue levels of monoamines in the rat. PLoS One. 2014 Jun 4;9(6):e98952.

Charvériat M, Naus CC, Leybaert L, Saez JC, Giaume C. Connexin-Dependent Neuroglial Networking as a New Therapeutic Target. Front Cell Neurosci. 2017 Jun 26;11:174.

Crossgrove J, Zheng W. Manganese toxicity upon overexposure. NMR Biomed. 2004 Dec;17(8):544-53.

Dhein S., Cardiovascular Research, 2004 (62) 287-298

Duchêne A, Perier M, Zhao Y, Liu X, Thomasson J, Chauveau F, Piérard C, Lagarde D, Picoli C, Jeanson T, Mouthon F, Dauvilliers Y, Giaume C, Lin JS, Charvériat M. Impact of Astroglial Connexins on Modafinil Pharmacological Properties. Sleep. 2016 Jun 1;39(6):1283-92.

Fawwaz RA, Hemphill W, Winchell HS. Potential use of Pd-porphyrin complexes for selective lymphatic ablation. J Nucl Med. 1971;12:231-236

Fujimori H, Matsumura A, Yamamoto T, Shibata Y, Yoshizawa T, Nakagawa K, Yoshii Y, Nose T, Sakata I, Nakajima S. Tumor specific contrast enhancement study of Mn-metalloporphyrin (ATN-10)-comparison of rat brain tumor model, cytotoxic and vasogenic edema models. Acta Neurochir. 1997;70:167-169.

Gale EM, Atanasova IP, Blasi F, Ay I, Caravan P. A Manganese Alternative to Gadolinium for MRI Contrast. J Am Chem Soc. 2015 Dec 16;137(49):15548-57.

Harks EG, The Journal of Pharmacology and Experimental Therapeutics 2001 Sep, 298(3): 1033-41

Huang LR, Straubinger RM, Kahl SB, Koo MS, Alletto JJ, Mazurchuk R, Chau RI, Thamer SL, Fiel RJ. Boronated metalloporphyrins: a novel approach to the diagnosis and treatment of cancer using contrast-enhanced MR imaging and neutron capture therapy. J Magn Reson Imaging. 1993 Mar-Apr;3(2):351-6.

Jeanson T, Duchêne A, Richard D, Bourgoin S, Picoli C, Ezan P, Mouthon F, Giaume C, Hamon M, Charvériat M. Potentiation of Amitriptyline Anti-Hyperalgesic-Like Action By Astroglial Connexin 43 Inhibition in Neuropathic Rats. Sci Rep. 2016 Dec 12;6:38766.

Jørgensen JT, Rief M, Brismar TB, Wagner M, Albiin N. A new manganese-based oral contrast agent (CMC-001) for liver MRI: pharmacological and pharmaceutical aspects. Acta Radiol. 2012 Sep 1;53(7):707-13.

Karlsson JO, Ignarro LJ, Lundström I, Jynge P, Almén T. Calmangafodipir [Ca4Mn(DPDP)5], mangafodipir (MnDPDP) and MnPLED with special reference to their SOD mimetic and therapeutic properties. Drug Discov Today. 2015 Apr;20(4):411-21.

Kosmacek EA et al MnTnBuOE-2-PyP protects normal colorectal fibroblasts from radiation damage and simultaneously enhances radio/chemotherapeutic killing of colorectal cancer cells. Oncotarget. 2016 Jun 7;7(23):34532-45.

Leung KC, Wang YX, Wang H, Xuan S, Chak CP, Cheng CH. Biological and magnetic contrast evaluation of shape-selective Mn-Fe nanowires. IEEE Trans Nanobiosci. 2009;2:192-198.

Leybaert L, Lampe PD, Dhein S, Kwak BR, Ferdinandy P, Beyer EC, Laird DW, Naus CC, Green CR, Schulz R. Connexins in Cardiovascular and Neurovascular Health and Disease: Pharmacological Implications. Pharmacol Rev. 2017 Oct;69(4):396-478.

Li D, Agulhon C, Schmidt E, Oheim M, Ropert N. New tools for investigating astrocyte-to-neuron communication. Front Cell Neurosci. 2013 Oct 29;7:193.

Li Z, Li W, Li X, Pei F, Wang X, Lei H. Mn(II)-monosubstituted polyoxometalates as candidates for contrast agents in magnetic resonance imaging. J Inorg Biochem. 2007;101:1036-1042

Louis DN, Perry A, Reifenberger G, von Deimling A, Figarella-Branger D, Cavenee WK, Ohgaki H, Wiestler OD, Kleihues P, Ellison DW. The 2016 World Health Organization Classification of Tumors of the Central Nervous System: a summary. Acta Neuropathol. 2016 Jun;131(6):803-20

Lu J, Ma S, Sun J, Xia C, Liu C, Wang Z, Zhao X, Gao F, Gong Q, Song B, Shuai X, Ai H, Gu Z. Manganese ferrite nanoparticle micellar nanocomposites as MRI contrast agent for liver imaging. Biomaterials. 2009;30:2919-2928

Malheiros et al, Manganese-Enhanced MRI: Biological Applications in Neuroscience. Front Neurol. 2015 Jul 10;6:161.

Massaad CA, Pautler RG. Manganese-enhanced magnetic resonance imaging (MEMRI). Methods Mol Biol. 2011;711:145-74.

Matsumura A1, Shibata Y, Yamamoto T, Nakagawa K, Yasuda S, Nakajima S, Sakata I, Yoshizawa T, Nose T. Mn-metalloporphyrin conjugated with Gd-DTPA (Gd-ATN10): tumor enhancement agent for magnetic resonance imaging. Neurol Med Chir (Tokyo). 1997 Apr;37(4):327-31.

Mertzman JE, Kar S, Lofland S, Fleming T, Keuren EV, Tong YY, Stoll SL. Surface attached manganese-oxo clusters as potential contrast agents. Chem Commun. 2009;45:788-790.

Na HB, Lee JH, An K, Park YI, Park M, Lee IS, Nam DH, Kim ST, Kim SH, Kim SW, Lim KH, Kim KS, Kim SO, Hyeon T. Development of a T1 contrast agent for magnetic resonance imaging using MnO nanoparticles. Angew Chem Int Ed Engl. 2007;46:5397-5401.

Ni Y, Petré C, Miao Y, Yu J, Cresens E, Adriaens P, Bosmans H, Semmler W, Baert AL, Marchal G; Magnetic resonance imaging-histomorphologic correlation studies on paramagnetic metalloporphyrins in rat models of necrosis. Invest Radiol. 1997 Dec;32(12):770-9.

Pan D, Senpan A, Caruthers SD, Williams TA, Scott MJ, Gaffney PJ, Wickline SA, Lanza GM. Sensitive and efficient detection of thrombus with fibrin-specific manganese nanocolloids. Chem Commun (Camb) 2009:3234-3236

Pan D, Caruthers SD, Hu G, Senpan A, Scott MJ, Gaffney PJ, Wickline SA, Lanza GM. Ligand-directed nanobialys as theranostic agent for drug delivery and manganese-based magnetic resonance imaging of vascular targets. J Am Chem Soc. 2008;130:9186-9187

Picoli C, Nouvel V, Aubry F, Reboul M, Duchêne A, Jeanson T, Thomasson J, Mouthon F, Charvériat M. Human connexin channel specificity of classical and new gap junction inhibitors. J Biomol Screen. 2012 Dec;17(10):1339-47.

Place DA, Faustino PJ, Berghmans KK, van Zijl PCM, Chesnick AS, Cohen JS. Magn Reson Imaging. 1992;10:919-928

Rongved P, Klaveness J. Water-soluble polysaccharides as carriers of paramagnetic contrast agents for magnetic resonance imaging: synthesis and relaxation properties. Carbohydr Res. 1991;214:315-323.

Rouach N1, Koulakoff A, Abudara V, Willecke K, Giaume C. Astroglial metabolic networks sustain hippocampal synaptic transmission. Science. 2008 Dec 5;322(5907):1551-5.

Salameh A, Biochimica et Biophysica Acta 1719 (2005) 36-58

Scadeng M. , D. J. Dubowitz, N. Gray, and E. Breen, MANGANESE TRACT TRACING IN ZEBRAFISH, Proc. Intl. Soc. Mag. Reson. Med. 17 (2009)

Schmiedl UP, Nelson JA, Robinson DH, Michalson A, Starr F, Frenzel T, Ebert W, Schuhmann-Giampieri G. Invest Radiol. 1993;28:925-932

Schwendener RA, Wüthrich R, Duewell S, Wehrli E, von Schulthess GK. A pharmacokinetic and MRI study of unilamellar gadolinium-, manganese-, and iron-DTPA-stearate liposomes as organ-specific contrast agents. Invest Radiol. 1990;25:922-932

Shapiro EM, Koretsky AP. Convertible manganese contrast for molecular and cellular MRI. Magn Reson Med. 2008;60:265-269

Shin SW et al., Mechanism of the Antitumor and Radiosensitizing Effects of a Manganese Porphyrin, MnHex-2-PyP. Antioxid Redox Signal. 2017 Nov 10;27(14):1067-1082. doi: 10.1089/ars.2016.6889.

Shin J, Anisur RM, Ko MK, Im GH, Lee JH, Lee IS. Hollow manganese oxide nanoparticles as multifunctional agents for magnetic resonance imaging and drug delivery. Angew Chem Int Ed Engl. 2009;48:321-324

Srivinas M, Connexins: A Guide, Humana Press 2009, Chapter 8, pages 207-224 Srinivas M, Molecular Pharmacology 2003 Jun, 63(6): 1389-97

Steibel BA, Michou-Gallani AI, Gallani JL, Felder-Flesch D. Development of a dendritic manganese-enhanced magnetic resonance imaging (MEMRI) contrast agent: synthesis, toxicity (in vitro) and relaxivity (in vitro, in vivo) studies. Bioconjug Chem. 2009;20:760-767

Takehara Y, Sakahara H, Masunaga H, Isogai S, Kodaira N, Sugiyama M, Takeda H, Saga T, Nakajima S, Sakata I. Assessment of a potential tumor-seeking manganese metalloporphyrin contrast agent in a mouse model. Magn Reson Med. 2002;47:549-553

Taylor KM, Rieter WJ, Lin W. Manganese-based nanoscale metal-organic frameworks for magnetic resonance imaging. J Am Chem Soc. 2008;130:14358-14359

Troughton JS, Greenfield MT, Greenwood JM, Dumas S, Wiethoff AJ, Wang J, Spiller M, McMury TJ, Caravan P. Synthesis and evaluation of a high relaxivity manganese(II)-based mri contrast agent. Inorg Chem. 2004;43:6313-6323

Unger E, Fritz T, Shen DK, Wu G. Manganese-based liposomes: comparative approach. Invest Radiol. 1993;28:933-938.

Watanabe T, Natt O, Boretius S, Frahm J, Michaelis T. In vivo 3D MRI staining of mouse brain after subcutaneous application of MnCl2. Magn Reson Med. 2002 Nov;48(5):852-9.

Wen PY, Kesari S. Malignant gliomas in adults. N Engl J Med. 2008 Jul 31;359(5):492-507.

Yang J, Lee CH, Ko HJ, Suh JS, Yoon HG, Lee K, Huh YM, Haam S. Multifunctional magneto-polymeric nanohybrids for targeted detection and synergistic therapeutic effects on breast cancer. Angew Chem Int Ed Engl. 2007;46:8836-8839

Zhao DW, Cheng C, Kuang LQ, Zhang YL, Cheng HY, Min JY, Wang Y. A New Approach Using Manganese-Enhanced MRI to Diagnose Acute Mesenteric Ischemia in a Rabbit Model: Initial Experience. Biomed Res Int. 2015;2015:579639.

**Claims**

1. Use of the MEMRI imaging technology to study the impact on the brain of a tested subject of candidate molecules that have connexin modulating activity.

2. Use according to claim 1, wherein said candidate molecule is chosen in the group consisting of: flecainide, long-chain alcohols, fenamates (such as meclofenamic acid, fenamic acid, mefenamic acid, niflumic acid, etofenamate, flufenamic acid), arylaminobenzoates, aminosulfonates, glycyrrhetinic acid derivatives, oleamides, or tetraalkylammonium ions and polyamines, quinine derivatives (such as quinine, mefloquine, quinidine), 2-ABP, anesthetic agents, cyclodextrins, antibodies directed against the extracellular domain of the connexins or peptides with conserved patterns mimicking this particular domain (in particular Gap26, Gap27, peptide 5, Gap19, L2, JM2, CT9, AAP10 peptides), oleic acid, palmitoleic acid, decaenoic acid, myristoleic acid, staurosporine, strophanthidin, ouabin, delta-9-tetrahydrocannabinol, 2-aminoethoxydiphenyl borate acetic acid, propionic acid, 12-O-tetradecanoylphorbol-13-acetate (TPA), 2,3 butandione monoxime, carbachol, noradrenaline, FGF-2, angiotensin-II, Atrial Natriuretic factor ANF, VEGF, 1-oleyl-2-acetyl-*sn*-glycerol, 11,12-epoxyeicosatrienoic acid, lidocaine, tonabersat (SB-220453, (cis-(-)-6-acetyl-4S-(3-chloro-4-fluorobenzoylamino)-3,4-dihydro-2,2-dimethyl-2H-1-benzopyran-3S-ol), Nexagon and Peptagon.

3. Use according to claim 1 or 2, wherein said tested subject is a laboratory animal.

4. Use according to anyone of claim 1 to 3, for monitoring a therapeutic treatment implying the administration of a connexin-blocking agent.

5. Use according to any one of claim 1 to 3, for diagnosing diseases implying connexin deregulation, preferably in children.

6. Use according to any one of claim 1 to 5, wherein a manganese-based compound is administered to said tested subject, wherein said compound is chosen in the group consisting of: manganese chloride tetrahydrate, Mn-(PyC3A), CMC-001, EVP 1001-1, calmangafodipir ($Ca_4Mn(DPDP)_5$), mangafodipir and MnPLED.

7. A method to study the neuron-glia networks in the brain, said method comprising analyzing the MEMRI signal of at least one region of the brain of a tested subject before and after the administration of a connexin-modulating agent.

8. The method of claim 7, wherein said connexin-modulating agent is a connexin-blocking agent, preferably a glial connexin-blocking agent.

9. The method of claim 7, wherein said Cx43-blocking agent is a shRNA inhibiting the Cx43 expression.

10. The method of any one of claims 7 to 9, wherein said tested subject is a healthy or a pathogenic non-human laboratory animal model.

11. The method of any one of claims 7 to 9, wherein said tested subject is a laboratory animal model suffering from a neurodegenerative disorder, a psychiatric disorder or a glioblastoma.

12. A combination product containing a connexin-blocking agent and a manganese-based compound for simultaneous, separated, or staggered use for treating patients suffering from cancer.

13. The combination product of claim 12, wherein it enhances IR or chemotherapeutic anti-tumor efficiency in said patients.

14. The combination product of claim 12 or 13, wherein said patient suffers from glioblastoma or astrocytoma.

15. The combination product of any one of claims 12 to 14, wherein said manganese-based compound is chosen in the group consisting of: manganese porphyrin, boronated manganese metalloporphyrin, and MnTnBuOE-2-PyP.

**Figure 1**

A (mefloquine)

## Figure 1 (continued)

B (Flecainide)

**Figure 1 (continued)**

C (MFA)

**EP 3 593 822 A1**

Europäisches Patentamt

European Patent Office

Office européen des brevets

# EUROPEAN SEARCH REPORT

Application Number

EP 18 30 5954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MALHEIROS JACKELINE MORAES ET AL: "Manganese-Enhanced MRI: Biological Applications in Neuroscience", FRONTIERS IN NEUROLOGY, FRONTIERS RESEARCH FOUNDATION, vol. 6, 10 July 2015 (2015-07-10), pages 161-1, XP009510255, ISSN: 1664-2295, DOI: 10.3389/FNEUR.2015.00161 * the whole document * | 1-11 | INV. A61K49/06 A61K33/32 A61K31/196 A61K31/445 A61K31/4709 A61P25/00 A61P35/00 |
| X | CN 102 551 710 A (WEST CHINA HOSPITAL OF SICHUAN UNIVERSITY; CHENGDU HUAXI HAIQI MEDICAL) 11 July 2012 (2012-07-11) * the whole document * | 1-11 | |
| X | JASON SMUCNY ET AL: "Functional magnetic resonance imaging of intrinsic brain networks for translational drug discovery", TRENDS IN PHARMACOLOGICAL SCIENCES., vol. 35, no. 8, 1 August 2014 (2014-08-01) , pages 397-403, XP055540374, GB ISSN: 0165-6147, DOI: 10.1016/j.tips.2014.05.001 * the whole document * | 1-11 | |
| | -/-- | | TECHNICAL FIELDS SEARCHED (IPC) A61K A61P |

The present search report has been drawn up for all claims

| Place of search | Date of completion of the search | Examiner |
|---|---|---|
| Munich | 18 January 2019 | Baumgärtner, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

21

Europäisches
Patentamt

European
Patent Office

Office européen
des brevets

# EUROPEAN SEARCH REPORT

Application Number

EP 18 30 5954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TREGELLAS J R ET AL: "Effects of an Alpha 7-Nicotinic Agonist on Default Network Activity in Schizophrenia", BIOLOGICAL PSYCHIATRY, ELSEVIER SCIENCE, NEW YORK, NY; US, vol. 69, no. 1, 1 January 2011 (2011-01-01), pages 7-11, XP027550066, ISSN: 0006-3223 [retrieved on 2010-08-21] * the whole document * ----- | 1-11 | |
| X | US 2014/121166 A1 (KIELIAN TAMMY [US]) 1 May 2014 (2014-05-01) * the whole document * ----- | 1-11 | |

TECHNICAL FIELDS
SEARCHED     (IPC)

The present search report has been drawn up for all claims

| Place of search | Date of completion of the search | Examiner |
|---|---|---|
| Munich | 18 January 2019 | Baumgärtner, Heike |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

Application Number

EP 18 30 5954

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-11

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 18 30 5954

The Search Division considers that the present European patent application does not comply with the requirements of unity of invention and relates to several inventions or groups of inventions, namely:

1. claims: 1-11

   Use of the MEMRI imaging technology to study the impact on the brain of a tested subject of candidate molecules that have connexin modulating activity
   A method to study the neuron-glia networks in the brain, said method comprising analyzing the MEMRI signal of at least one region of the brain of a tested subject before and after the administration of a connexin-modulating agent
   ---

2. claims: 12-15

   combination product containing a connexin-blocking agent and a manganese-based compound for simultaneous, separated, or staggered use for treating patients suffering from cancer
   ---

## ANNEX TO THE EUROPEAN SEARCH REPORT
## ON EUROPEAN PATENT APPLICATION NO.

EP 18 30 5954

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2019

| Patent document cited in search report | | Publication date | Patent family member(s) | | Publication date |
|---|---|---|---|---|---|
| CN 102551710 | A | 11-07-2012 | NONE | | |
| US 2014121166 | A1 | 01-05-2014 | US 2014121166 | A1 | 01-05-2014 |
| | | | US 2017049761 | A1 | 23-02-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010029131 A
- WO 2013064579 A
- WO 2015011246 A

### Non-patent literature cited in the description

- **PICOLI C et al.** *J. Biomol. Screen,* 2012
- **JEANSON T et al.** *Scientific reports,* 2016
- **PAXINOS ; FRANKLIN'S.** Mouse Brain in Stereotaxic Coordinates
- **AIME S ; ANELLI PL ; BOTTA M ; BROCCHETTA M ; CANTON S ; FEDELI F ; GIANOLIO E ; TERRENO E.** Relaxometric evaluation of novel manganese (II) complexes for application as contrast agents in magnetic resonance imaging. *J Biol Inorg Chem.,* 2002, vol. 7, 58-67
- **AOKI I ; NARUSE S ; TANAKA C.** Manganese-enhanced magnetic resonance imaging (MEMRI) of brain activity and applications to early detection of brain ischemia. *NMR Biomed.,* December 2004, vol. 17 (8), 569-80
- **ASCHNER M ; GUILARTE TR ; SCHNEIDER JS ; ZHENG W.** Manganese: recent advances in understanding its transport and neurotoxicity. *Toxicol Appl Pharmacol,* 01 June 2007, vol. 221 (2), 131-47
- **BERGHOFF AS ; PREUSSER M.** Anti-angiogenic therapies in brain metastases. *Memo.,* 2018, vol. 11 (1), 14-17
- **BOUABID S ; DELAVILLE C ; DE DEURWAERDÈRE P ; LAKHDAR-GHAZAL N ; BENAZZOUZ A.** Manganese-induced atypical parkinsonism is associated with altered Basal Ganglia activity and changes in tissue levels of monoamines in the rat. *PLoS One,* 04 June 2014, vol. 9 (6), e98952
- **CHARVÉRIAT M ; NAUS CC ; LEYBAERT L ; SAEZ JC ; GIAUME C.** Connexin-Dependent Neuroglial Networking as a New Therapeutic Target. *Front Cell Neurosci.,* 26 June 2017, vol. 11, 174
- **CROSSGROVE J ; ZHENG W.** Manganese toxicity upon overexposure. *NMR Biomed.,* December 2004, vol. 17 (8), 544-53
- **DHEIN S.** *Cardiovascular Research,* 2004, vol. 62, 287-298
- **DUCHÊNE A ; PERIER M ; ZHAO Y ; LIU X ; THOMASSON J ; CHAUVEAU F ; PIÉRARD C ; LAGARDE D ; PICOLI C ; JEANSON T.** Impact of Astroglial Connexins on Modafinil Pharmacological Properties. *Sleep,* 01 June 2016, vol. 39 (6), 1283-92
- **FAWWAZ RA ; HEMPHILL W ; WINCHELL HS.** Potential use of Pd-porphyrin complexes for selective lymphatic ablation. *J Nucl Med.,* 1971, vol. 12, 231-236
- **FUJIMORI H ; MATSUMURA A ; YAMAMOTO T ; SHIBATA Y ; YOSHIZAWA T ; NAKAGAWA K ; YOSHII Y ; NOSE T ; SAKATA I ; NAKAJIMA S.** Tumor specific contrast enhancement study of Mn-metalloporphyrin (ATN-10)-comparison of rat brain tumor model, cytotoxic and vasogenic edema models. *Acta Neurochir,* 1997, vol. 70, 167-169
- **GALE EM ; ATANASOVA IP ; BLASI F ; AY I ; CARAVAN P.** A Manganese Alternative to Gadolinium for MRI Contrast. *J Am Chem Soc.,* 16 December 2015, vol. 137 (49), 15548-57
- **HARKS EG.** *The Journal of Pharmacology and Experimental Therapeutics,* 29 September 2001, vol. 8 (3), 1033-41
- **HUANG LR ; STRAUBINGER RM ; KAHL SB ; KOO MS ; ALLETTO JJ ; MAZURCHUK R ; CHAU RI ; THAMER SL ; FIEL RJ.** Boronated metalloporphyrins: a novel approach to the diagnosis and treatment of cancer using contrast-enhanced MR imaging and neutron capture therapy. *J Magn Reson Imaging,* March 1993, vol. 3 (2), 351-6
- **JEANSON T ; DUCHÊNE A ; RICHARD D ; BOURGOIN S ; PICOLI C ; EZAN P ; MOUTHON F ; GIAUME C ; HAMON M ; CHARVÉRIAT M.** Potentiation of Amitriptyline Anti-Hyperalgesic-Like Action By Astroglial Connexin 43 Inhibition in Neuropathic Rats. *Sci Rep.,* 12 December 2016, vol. 6, 38766
- **JØRGENSEN JT ; RIEF M ; BRISMAR TB ; WAGNER M ; ALBIIN N.** A new manganese-based oral contrast agent (CMC-001) for liver MRI: pharmacological and pharmaceutical aspects. *Acta Radiol.,* 01 September 2012, vol. 53 (7), 707-13

- **KARLSSON JO ; IGNARRO LJ ; LUNDSTRÖM I ; JYNGE P ; ALMÉN T.** Calmangafodipir [Ca4Mn(DP-DP)5], mangafodipir (MnDPDP) and MnPLED with special reference to their SOD mimetic and therapeutic properties. *Drug Discov Today,* April 2015, vol. 20 (4), 411-21
- **KOSMACEK EA et al.** MnTnBuOE-2-PyP protects normal colorectal fibroblasts from radiation damage and simultaneously enhances radio/chemotherapeutic killing of colorectal cancer cells. *Oncotarget,* 07 June 2016, vol. 7 (23), 34532-45
- **LEUNG KC ; WANG YX ; WANG H ; XUAN S ; CHAK CP ; CHENG CH.** Biological and magnetic contrast evaluation of shape-selective Mn-Fe nanowires. *IEEE Trans Nanobiosci.,* 2009, vol. 2, 192-198
- **LEYBAERT L ; LAMPE PD ; DHEIN S ; KWAK BR ; FERDINANDY P ; BEYER EC ; LAIRD DW ; NAUS CC ; GREEN CR ; SCHULZ R.** Connexins in Cardiovascular and Neurovascular Health and Disease: Pharmacological Implications. *Pharmacol Rev.,* October 2017, vol. 69 (4), 396-478
- **LI D ; AGULHON C ; SCHMIDT E ; OHEIM M ; ROPERT N.** New tools for investigating astrocyte-to-neuron communication. *Front Cell Neurosci.,* 29 October 2013, vol. 7, 193
- **LI Z ; LI W ; LI X ; PEI F ; WANG X ; LEI H.** Mn(II)-monosubstituted polyoxometalates as candidates for contrast agents in magnetic resonance imaging. *J Inorg Biochem.,* 2007, vol. 101, 1036-1042
- **LOUIS DN ; PERRY A ; REIFENBERGER G ; VON DEIMLING A ; FIGARELLA-BRANGER D ; CAVENEE WK ; OHGAKI H ; WIESTLER OD ; KLEIHUES P ; ELLISON DW.** The 2016 World Health Organization Classification of Tumors of the Central Nervous System: a summary. *Acta Neuropathol.,* June 2016, vol. 131 (6), 803-20
- **LU J ; MA S ; SUN J ; XIA C ; LIU C ; WANG Z ; ZHAO X ; GAO F ; GONG Q ; SONG B.** Manganese ferrite nanoparticle micellar nanocomposites as MRI contrast agent for liver imaging. *Biomaterials,* 2009, vol. 30, 2919-2928
- **MALHEIROS et al.** Manganese-Enhanced MRI: Biological Applications in Neuroscience. *Front Neurol.,* 10 July 2015, vol. 6, 161
- **MASSAAD CA ; PAUTLER RG.** Manganese-enhanced magnetic resonance imaging (MEMRI). *Methods Mol Biol.,* 2011, vol. 711, 145-74
- **MATSUMURA A1 ; SHIBATA Y ; YAMAMOTO T ; NAKAGAWA K ; YASUDA S ; NAKAJIMA S ; SAKATA I ; YOSHIZAWA T ; NOSE T.** Mn-metalloporphyrin conjugated with Gd-DTPA (Gd-ATN10): tumor enhancement agent for magnetic resonance imaging. *Neurol Med Chir (Tokyo),* April 1997, vol. 37 (4), 327-31
- **MERTZMAN JE ; KAR S ; LOFLAND S ; FLEMING T ; KEUREN EV ; TONG YY ; STOLL SL.** Surface attached manganese-oxo clusters as potential contrast agents. *Chem Commun.,* 2009, vol. 45, 788-790
- **NA HB ; LEE JH ; AN K ; PARK YI ; PARK M ; LEE IS ; NAM DH ; KIM ST ; KIM SH ; KIM SW.** Development of a T1 contrast agent for magnetic resonance imaging using MnO nanoparticles. *Angew Chem Int Ed Engl.,* 2007, vol. 46, 5397-5401
- **NI Y ; PETRÉ C ; MIAO Y ; YU J ; CRESENS E ; ADRIAENS P ; BOSMANS H ; SEMMLER W ; BAERT AL ; MARCHAL G.** Magnetic resonance imaging-histomorphologic correlation studies on paramagnetic metalloporphyrins in rat models of necrosis. *Invest Radiol.,* December 1997, vol. 32 (12), 770-9
- **PAN D ; SENPAN A ; CARUTHERS SD ; WILLIAMS TA ; SCOTT MJ ; GAFFNEY PJ ; WICKLINE SA ; LANZA GM.** Sensitive and efficient detection of thrombus with fibrin-specific manganese nanocolloids. *Chem Commun (Camb),* 2009, 3234-3236
- **PAN D ; CARUTHERS SD ; HU G ; SENPAN A ; SCOTT MJ ; GAFFNEY PJ ; WICKLINE SA ; LANZA GM.** Ligand-directed nanobialys as theranostic agent for drug delivery and manganese-based magnetic resonance imaging of vascular targets. *J Am Chem Soc.,* 2008, vol. 130, 9186-9187
- **PICOLI C ; NOUVEL V ; AUBRY F ; REBOUL M ; DUCHÊNE A ; JEANSON T ; THOMASSON J ; MOUTHON F ; CHARVÉRIAT M.** Human connexin channel specificity of classical and new gap junction inhibitors. *J Biomol Screen.,* December 2012, vol. 17 (10), 1339-47
- **PLACE DA ; FAUSTINO PJ ; BERGHMANS KK ; VAN ZIJL PCM ; CHESNICK AS ; COHEN JS.** *Magn Reson Imaging,* 1992, vol. 10, 919-928
- **RONGVED P ; KLAVENESS J.** Water-soluble polysaccharides as carriers of paramagnetic contrast agents for magnetic resonance imaging: synthesis and relaxation properties. *Carbohydr Res.,* 1991, vol. 214, 315-323
- **ROUACH N1 ; KOULAKOFF A ; ABUDARA V ; WILLECKE K ; GIAUME C.** Astroglial metabolic networks sustain hippocampal synaptic transmission. *Science,* 05 December 2008, vol. 322 (5907), 1551-5
- **SALAMEH A.** *Biochimica et Biophysica Acta,* 2005, vol. 1719, 36-58
- **SCADENG M. ; D. J. DUBOWITZ ; N. GRAY ; E. BREEN.** MANGANESE TRACT TRACING IN ZEBRAFISH. *Proc. Intl. Soc. Mag. Reson. Med.,* 2009, vol. 17
- **SCHMIEDL UP ; NELSON JA ; ROBINSON DH ; MICHALSON A ; STARR F ; FRENZEL T ; EBERT W ; SCHUHMANN-GIAMPIERI G.** *Invest Radiol.,* 1993, vol. 28, 925-932

- **SCHWENDENER RA ; WÜTHRICH R ; DUEWELL S ; WEHRLI E ; VON SCHULTHESS GK.** A pharmacokinetic and MRI study of unilamellar gadolinium-, manganese-, and iron-DTPA-stearate liposomes as organ-specific contrast agents. *Invest Radiol.,* 1990, vol. 25, 922-932
- **SHAPIRO EM ; KORETSKY AP.** Convertible manganese contrast for molecular and cellular MRI. *Magn Reson Med.,* 2008, vol. 60, 265-269
- **SHIN SW et al.** Mechanism of the Antitumor and Radiosensitizing Effects of a Manganese Porphyrin, Mn-Hex-2-PyP. *Antioxid Redox Signal,* 10 November 2017, vol. 27 (14), 1067-1082
- **SHIN J ; ANISUR RM ; KO MK ; IM GH ; LEE JH ; LEE IS.** Hollow manganese oxide nanoparticles as multifunctional agents for magnetic resonance imaging and drug delivery. *Angew Chem Int Ed Engl.,* 2009, vol. 48, 321-324
- **SRIVINAS M.** Connexins: A Guide. Humana Press, 2009, 207-224
- **SRINIVAS M.** *Molecular Pharmacology,* June 2003, vol. 63 (6), 1389-97
- **STEIBEL BA ; MICHOU-GALLANI AL ; GALLANI JL ; FELDER-FLESCH D.** Development of a dendritic manganese-enhanced magnetic resonance imaging (MEMRI) contrast agent: synthesis, toxicity (in vitro) and relaxivity (in vitro, in vivo) studies. *Bioconjug Chem.,* 2009, vol. 20, 760-767
- **TAKEHARA Y ; SAKAHARA H ; MASUNAGA H ; ISOGAI S ; KODAIRA N ; SUGIYAMA M ; TAKEDA H ; SAGA T ; NAKAJIMA S ; SAKATA I.** Assessment of a potential tumor-seeking manganese metalloporphyrin contrast agent in a mouse model. *Magn Reson Med.,* 2002, vol. 47, 549-553
- **TAYLOR KM ; RIETER WJ ; LIN W.** Manganese-based nanoscale metal-organic frameworks for magnetic resonance imaging. *J Am Chem Soc.,* 2008, vol. 130, 14358-14359
- **TROUGHTON JS ; GREENFIELD MT ; GREENWOOD JM ; DUMAS S ; WIETHOFF AJ ; WANG J ; SPILLER M ; MCMURY TJ ; CARAVAN P.** Synthesis and evaluation of a high relaxivity manganese(II)-based mri contrast agent. *Inorg Chem.,* 2004, vol. 43, 6313-6323
- **UNGER E ; FRITZ T ; SHEN DK ; WU G.** Manganese-based liposomes: comparative approach. *Invest Radiol.,* 1993, vol. 28, 933-938
- **WATANABE T ; NATT O ; BORETIUS S ; FRAHM J ; MICHAELIS T.** In vivo 3D MRI staining of mouse brain after subcutaneous application of MnCl2. *Magn Reson Med.,* November 2002, vol. 48 (5), 852-9
- **WEN PY ; KESARI S.** Malignant gliomas in adults. *N Engl J Med.,* 31 July 2008, vol. 359 (5), 492-507
- **YANG J ; LEE CH ; KO HJ ; SUH JS ; YOON HG ; LEE K ; HUH YM ; HAAM S.** Multifunctional magneto-polymeric nanohybrids for targeted detection and synergistic therapeutic effects on breast cancer. *Angew Chem Int Ed Engl.,* 2007, vol. 46, 8836-8839
- **ZHAO DW ; CHENG C ; KUANG LQ ; ZHANG YL ; CHENG HY ; MIN JY ; WANG Y.** A New Approach Using Manganese-Enhanced MRI to Diagnose Acute Mesenteric Ischemia in a Rabbit Model: Initial Experience. *Biomed Res Int.,* 2015, vol. 2015, 579639